# EUROPEAN PATENT APPLICATION

(11) **EP 0 687 466 A1**
(43) Date of publication of application: **20.12.1995**
(21) Application number: 95304210.8
(22) Date of filing: 16.06.1995
(51) Int. Cl.: A61K 31/00, A61K 9/28

(54) **Homeopathic formulations**

(30) Priority: 16.06.1994 IL 11003594
(71) Applicant: TAPUACH NATURAL TECHNOLOGIES (1993) LTD., Ramat Hasharon 47227 (IL)
(72) Inventor: Amitai, Gabriel, Rehovot 76281 (IL); Borenstein, Amiram, Rishon Lezion 75436 (IL)
(74) Representative: Goldin, Douglas Michael

(57) **Abstract**

A slow or controlled release particulate solid formulation for use in homeopathic medicine, which comprises at least one medicament and at least one polymerized sugar.

## Description

### FIELD AND BACKGROUND OF THE INVENTION

The present invention relates to slow and controlled release particulate formulations for administration of homeopathic medicaments.

Homeopathic medicine involves administration of medicaments in amounts below levels which are effective according to conventional pharmaceutical and pharmocological principles, as well as medicaments which are not considered as lying within the sphere of conventional medical practice. Homeopathic medicaments will usually contain the active ingredient in a concentration within the range 10⁻¹² to 10⁻¹ (e.g. 10⁻¹⁰ to 10⁻³ or 10⁻²) wt. %. It is usual in homeopathic medicine to administer doses of medicaments repeatedly at intervals which can vary from a few minutes or hours, to days, weeks or months. It would be useful to be able to administer a single dose of homeopathic medicament such that it would become available in the human body over a prolonged period of time (without repeated administration), but homeopathic medicaments of this nature have not been generally available hitherto, to the best of the inventors' knowledge. One of the objects of the present invention is to remedy this deficiency in the homeopathic practitioner's pharmacopeia. Other objects of the invention will appear from the description herein.

It will be apparent from what has been said above, that the term "medicament" as used in the present specification and claims is intended to include both conventional pharmacologically active substances in amounts below levels which are effective according to conventional pharmaceutical and pharmocological principles, as well as substances used according to homeopathic practice but which are not generally considered therapeutically active (or may even be toxic at certain dosages) according to conventional (non-homeopathic) practice.

### SUMMARY OF THE INVENTION

In one aspect, the present invention provides a slow or controlled release particulate solid formulation for use in homeopathic medicine, which comprises at least one medicament and at least one polymerized sugar. In a particular embodiment, the at least one medicament is supported on an inert carrier, the inert carrier being selected from inert carriers which have not been known in the art as providing slow or controlled release characteristics.

The at least one polymerized sugar may have been incorporated in the formulation in one of the following modes (a), (b) and (c), namely: (a) by coating at least part of the carrier (when present); (b) by coating at least part of the at least one medicament; (c) by coating at least part of the carrier (when present), as well as at least part of the at least one medicament, and preferably by coating at least part of the supported medicament as a whole.

### DETAILED DESCRIPTION OF THE INVENTION

The active ingredient or medicament may be supported on an inert carrier. The inert carrier is generally not one which is known to provide slow or controlled release characteristics. The inert carrier may be either water-soluble or insoluble. Insoluble carriers are those known in pharmaceutical or homeopathic practice; soluble carriers may be e.g. non-polymerized sugars such as lactose and sucrose. The polymerized sugar may be for example, chitin, chitosan (deacetylated chitin), cellulose, alkylated celluloses such as methyl, ethyl and hydroxypropyl celluloses, and alginic acid and alginates. Derivatives of polymerized sugars, e.g. esters such as the acetates may also be used, and the meaning of the term "polymerized sugar" as used in the present specification and claims should be understood accordingly. The polymerised sugar is preferably present in the form of a coating.

The thickness of the polymerised sugar coating may be varied in order to vary the rate of controlled release of medicament, a relatively thinner coating giving a relatively rapid release of medicament and a relatively thicker coating giving a relatively slow release of medicament.

When a carrier is used, the coating may be applied, preferably to the medicament before incorporation in the carrier, or more preferably to the supported medicament as a whole, by treating the medicament, or the supported medicament, as the case may be, with a solution or suspension of the polymerized sugar in a solvent medium such as water, acids or organic solvents. The skilled artisan will be capable of selecting the medium having regard to its solvent power both for the medicament and the support, and the stability of both medicament and support therein.

It will be appreciated that the particulate formulation may be used per se, i.e. in the form of powders, for oral administration, or may be compressed into tablets or used to fill capsules, for this purpose. Finely-divided powders may also be used for administration by insufflation, e.g. into the nasal passages.

The invention will be illustrated by the following non-limitative Examples, in which the products may be administered orally, unless stated otherwise.
(1) A solution in alcohol (2 ml) containing and 0.01% active ingredient (e.g. camphor, Belladonna extract or digitalis) is sprayed over 10 minutes at 40°C onto sugar beads (10 g, e.g. of sucrose or lactose) having a 1-5 mm range of diameter, in a laboratory pan-coating apparatus. Subsequently, a solution in alcohol (3 ml) containing 1% polymer (e.g. ethylcellulose, hydroxypropyl cellulose, or one of Eudragits L. E, S, or RS) is sprayed onto the rotated beads from the previous step, i.e. containing active ingredient, over 15 minutes at 40°C, while passing a gentle stream of air through the apparatus. The coated beads thus produced are free flowing and can be administered orally or packed into 250 or 500 mg size capsules. The duration and release profile of active ingredient is controlled by the amount of polymer solution sprayed, and on the concentration and the type of polymer.
   The slow release characteristics of the product were tested by preparing similarly the analogous product containing radiolabelled camphor (New England Nuclear, Boston) (10 mCi/g). 100 mg of the product is placed in 100 ml 0.1M phosphate buffer solution at pH 7.4, simulating physiological fluid. Samples (0.4 ml) are taken every two hours for 12 hours, centrifuged to remove solids, and the supernatant is tested for radioactivity. Radioactivity was found in all solutions tested, in increasing amounts, thus demonstrating slow release of the active ingredient over at least 12 hours.
(2) A solution in alcohol (2 ml) containing 1% polymer (e.g. as in the preceding Example) and 0.01% active ingredient (e.g. camphor, Belladonna extract or digitalis) is sprayed over 10 minutes at 40°C onto sugar beads of 1 mm diameter (10 g) in a laboratory pan-coating apparatus. Subsequently, a solution in alcohol (3 ml) containing 1% polymer (as previously identified) is sprayed onto the rotated beads from the previous step, i.e. containing active ingredient, over 15 minutes at 40°C, while passing a gentle stream of air through the apparatus. The coated beads thus produced are free flowing and can be administered orally or packed into 250 or 500 mg size capsules. The duration and release profile of active ingredient is controlled by the amount of polymer solution sprayed, and on the concentration and the type of polymer. The slow release characteristics of the product were tested by the technique described in the previous Example, and similar results were obtained.
(3) A solution of camphor (0.001% w/v) and ethylcellulose ("Ethocel", Dow, 1%) in ethanol is spray dried using a Bucht Spray Drier at 30°C. Fine microparticles in the 1-10 µ range are obtained. Similarly, higher or lower polymer concentrations may be used to provide a range of release rates of active ingredient. Methylcellulose, hydroxypropyl cellulose, or one of Eudragits L. E, S, or RS, may be used in place of the ethylcellulose. Acetone or acetone/ethanol may be used in place of ethanol. Any suitable active ingredient, such as belladonna extract, digitalis, phenol or benzoic acid may be substituted for the camphor. The product may be administered orally or as a spray for absorption in the respiratory tract.
(4) Calcium carbonate of approx. 1 µ particle size is dispersed in an amount of 0.001% in 5% (w/v) aqueous solution of sodium alginate and the dispersion is spray dried at 80°C, when a white powder having a particle size 5-20 µ is produced. The calcium carbonate may be replaced by calcium phosphate, sodium sulfate, ammonium carbonate, silica or alumina (each of which is in the form of particles of size approximately 1 µ), to give similar results.
(5) Active ingredient (5 mg) was mixed with mint, crystalline cellulose (5 g) and hydroxypropylcellulose (2 ml) and the mixture compressed into 1-5 mm pellets. Release of active compound is controlled by swelling of the pellets in gastric fluid over a period of 12 hours. The active ingredients may be, for example, silver nitrate, alumina, iron (ferrous or ferric) picrate, antimony potassium tartrate, or iron (ferrous or ferric) phosphate.

While particular embodiments of the invention have been described, it will be evident to persons skilled in the art that many variations and modifications may be made. The present invention is accordingly not to be construed as limited by the embodiments which have been particularly described, rather its concept, spirit and scope will be better appreciated by reference to the claims which follow.

## Claims

1. A slow or controlled release particulate solid formulation for use in homeopathic medicine which comprises at least one medicament and at least one polymerized sugar or derivative thereof.

2. A formulation according to claim 1, wherein the at least one medicament is supported on an inert carrier.

3. A formulation according to claim 1 or 2, wherein the at least one polymerized sugar is present in the formulation as a coating on at least part of the at least one medicament.

4. A formulation according to claim 2 or 3, wherein the at least one polymerized sugar is present in the formulation as a coating on at least part of the carrier.

5. A formulation according to claim 2, 3 or 4 wherein the at least one polymerized sugar is present in the formulation as a coating on at least part of the supported medicament.

6. A formulation according to any one of claims 2 to 5, wherein the inert carrier is water-insoluble.

7. A formulation according to any one of claims 2 to 5, wherein the inert carrier is water-soluble.

8. A formulation according to claim 7, wherein the inert carrier is at least one non-polymerized sugar.

9. A formulation according to claim 8, wherein the at least one non-polymerized sugar is lactose or sucrose.

10. A formulation according to any one of the preceding claims, wherein the polymerized sugar or derivative thereof is selected from chitin, chitosan, cellulose, alkylated cellulose, alginic acid, alginates, esters of polymerized sugars and mixtures thereof.

11. A process for preparing a formulation as claimed in any one of the preceding claims which process comprises contacting at least one medicament with at least one polymerized sugar or derivative thereof.
